# EUROPÄISCHE PATENTANMELDUNG

(11) **EP 2 264 449 A1**
(43) Veröffentlichungstag der Anmeldung: **22.12.2010**
(21) Anmeldenummer: 09162760.4
(22) Anmeldetag: 16.06.2009
(51) Int. Cl.: G01N 33/18

(54) **Fotometrisches Verfahren zur quantitativen Bestimmung eines Analyts in Wasser**

(71) Anmelder: Hach Lange GmbH, 14163 Berlin (DE)
(72) Erfinder: Farjam, Dr. Aria, 40223 Düsseldorf (DE); Bielefeld, Dr. Michael, 42113 Wuppertal (DE)
(74) Vertreter: Patentanwälte ter Smitten

(57) **Zusammenfassung**

Die Erfindung bezieht sich auf ein fotometrisches Verfahren zur quantitativen Bestimmung eines Analyts in Wasser, mit den Verfahrensschritten:
Erzeugen einer das Anolyt enthaltenden Flüssigkeitsprobe,
Hinzugeben eines Reagenzes zu der Flüssigkeitsprobe, wobei das Reagenz bei einer Reagenz-Absorptionswellenlänge λ_{R} farbverändernd mit dem Analyt reagiert, und
Fotometrieren der Flüssigkeitsprobe in einem Fotometer bei der Reagenz-Absorptionswellenlänge λ_{R},
Fotometrieren der Flüssigkeitsprobe mit dem Fotometer bei der Farbstandard- Absorptionswellenlänge λ_{F}, und
Ermitteln der fotometrischen Absorption der Flüssigkeitsprobe bei der Farbstandard- Absorptionswellenlänge λ_{F}.
der dem Reagenz zugefügte inerte Farbstandard weist eine bekannte Konzentration auf, wobei der Farbstandard eine charakteristische Absorbtionswellenlänge λ_{F} aufweist und das Reagenz-Absorbtionsspektrum und das Farbstandard- Absorbtionsspektrum so weit auseinander liegen, dass sie sich bei den jeweiligen Absorbtionswellenlängen λ_{R}, λ_{F} nicht überlagern.

## Beschreibung

Die Erfindung bezieht sich auf ein fotometrisches Verfahren zur quantitativen Bestimmung eines Analyts in Wasser.

Die vorliegende Erfindung bezieht sich sowohl auf die Laboranalytik als auch auf die Prozessanalytik zur quantitativen Bestimmung eines Analyts in Wasser, insbesondere in Abwasser, Trinkwasser, Wasser in Zuchtbecken etc.

Beim laboranalytischen Vorgehen wird zunächst eine Wasserprobe vorgegebener Menge mit Hilfe einer Pipette aus dem Wasser in ein kleines Gefäß, beispielsweise in eine Küvette gegeben. In der Regel befindet sich in dem Gefäß bereits das flüssige, in Ausnahmefällen feste, Reagenz in einer definierten Menge, oder aber wird anschließend hinzugegeben. Das Reagenz reagiert bei einer Absorptionswellenlänge farbverändernd mit dem Analyt. Mit einem Labor-Fotometer wird diese Farbveränderung quantitativ bestimmt, und hieraus die Konzentration beziehungsweise die Menge des Analyts in der Probe ermittelt und von dem Fotometer ausgegeben. Dieser Rückschluss auf die Konzentration beziehungsweise die Menge an Analyt in der Probe setzt jedoch unter anderem voraus, dass das Mengenverhältnis von Probe und Reagenz bekannt beziehungsweise immer gleich ist. Beim Pipettieren des Wassers und gegebenenfalls beim Dosieren des Reagenzes können jedoch Mengen-Fehler auftreten, die nicht ohne weiteres bemerkt werden können, jedoch wegen der sich hierdurch ändernden Verdünnung der Probe durch das Anolyt oder wegen der durch den fehlenden Reagenz-Überschusses verursachten unvollständigen Reaktion zu falschen Rückschlüsseln auf die Konzentration führen.

Beim prozessanalytischen Vorgehen, bei dem die Probennahme und die Dosierung des Analyts in der Regel vollautomatisch erfolgen, können ebenfalls Fehler bei der mengenmäßigen Dosierung der Probe und des Analyts auftreten, die nicht ohne weiteres bemerkt werden können. Darüber hinaus können Luftblasen in der Proben- Flüssigkeit, während diese durch die Fotometer-Messstrecke fließt und fotometriert wird, das Messergebnis erheblich verfälschen.

Aufgabe der Erfindung ist es demgegenüber, ein fotometrisches Verfahren zur fehlerfreien quantitativen Bestimmung eines Analyts in Wasser zu schaffen.

Diese Aufgabe wird erfindungsgemäß gelöst durch ein fotometrisches Verfahren mit den Merkmalen des Patentanspruches 1.

Bei dem erfindungsgemäßen fotometrischen Verfahren ist dem Reagenz ein inerter Farbstandard in bekannter Konzentration bzw. Menge zugefügt, wobei der Farbstandard eine charakteristische Absorptionswellenlänge aufweist und das Reagenz-Absorptionsspektrum und das Farbstandard-Absorptionsspektrum so weit auseinander liegen, dass sie sich bei den jeweiligen Absorptionswellenlängen nicht relevant überlagern. Mit anderen Worten, das Reagenz ist mit einem Farbstoff homogen vermischt, wobei das Absorptionsspektrum des Farbstoffes nicht interferiert mit der Wellenlänge, bei der die Absorption des Reagenzes fotometrisch bestimmt wird. Der Farbstandard ist in Bezug auf das Anolyt inert, es treten also keinerlei chemische Wechselwirkungen zwischen dem Farbstandard und dem Analyt auf.

Die Flüssigkeitsprobe wird mit dem Fotometer bei der Farbstandard-Absorptionswellenlänge fotometriert. Mit dem Ergebnis dieser Fotometrie wird die fotometrische Absorption der Probe bei der Farbstandard-Absorptionswellenlänge durch das Fotometer ermittelt. Aus der auf diese Weise ermittelten Farbstandard-Absorption kann das genaue Mischungsverhältnis von Flüssigkeitsprobe und Reagenz ermittelt werden. Dies ist bei einem flüssigen Reagenz deshalb von großer Wichtigkeit, weil durch das Mischungsverhältnis beziehungsweise die Verbindung der Flüssigkeitsprobe durch das flüssige Reagenz das Ergebnis der Fotometrie bei der Reagenz-Absorptionswellenlänge unmittelbar beziehungsweise proportional beeinfiusst wird. Die Feststellung des genauen Mischungsverhältnisses ist insbesondere bei der Laboranalyse von großer praktischer Bedeutung, da hier die Dosierung der Flüssigkeitsprobe in der Regel manuell durch Pipettieren erfolgt, und daher schwanken kann.

Auch für den Fall, dass das Reagenz ohne die Flüssigkeitsprobe bei der Reagenz-Absorptionswellenlänge bereits absorbiert, also einen Offset bei der Reagenz-Absorptionswellenlänge aufweist, muss das genaue Mischungsverhältnis bekannt sein. Nur wenn das genaue Mischungsverhältnis bekannt ist, kann von der Gesamt-Absorption der Flüssigkeitsprobe bei der Reagenz-Absorptionswellenlänge die Offset-Absorption des Reagenzes korrekt abgezogen werden, um die durch das Analyt begründete Absorption und damit seine Konzentration in der Wasserprobe genau bestimmen zu können.

Bei der prozessanalytischen Fotometrie kann davon ausgegangen werden, dass die mengenmäßige Dosierung der Flüssigkeitsprobe und des flüssigen Analyts in der Regel innerhalb gewisser Toleranzen funktioniert, also das Mischungsverhältnis der Flüssigkeitsprobe und des flüssigen Analyts nicht sehr stark schwankt. Unter dieser Voraussetzung stellt sich in der Fotometer-Messstrecke in Bezug auf die Farbstandard-Absorptionswellenlänge stets eine Absorption innerhalb eines kleineren Intervalls ein. Ist dies nicht der Fall, und ist die ermittelte Absorption insbesondere erheblich geringer als der Sollwert, kann von einer oder mehreren Luftblasen im Bereich der Messstrecke oder aber von einer erheblichen Abweichung des Mischungsverhältnisses ausgegangen werden. Die korrespondierende Messung der Reagenz-Absorption kann in diesem Fall dann verworfen werden.

Bei einem nicht-flüssigen Reagenz, das beispielsweise im Pulverform vorliegt, kann durch die Auswertung des Ergebnisses der Fotometrie bei der Farbstandard-Absorptionswellenlänge sichergestellt werden, dass das Reagenz in einem ausreichenden Überschuss vorliegt, so dass sichergestellt werden kann, dass das Anolyt in der Flüssigkeitsprobe vollständig mit dem Reagenz reagiert.

Vorzugsweise wird die Flüssigkeitsprobe durch Isolieren einer Wasserprobe aus dem Wasser, beispielsweise aus Abwasser oder Trinkwasser, in ein Gefäß erzeugt. Dies ist das Vorgehen bei der Laboranalyse, bei der die Wasserprobe aus dem Wasser üblicherweise in eine Küvette pipettiert wird, in der sich bereits das flüssige Reagenz befindet.

Alternativ hierzu kann die Flüssigkeitsprobe durch Dialyse des Analyts aus dem Wasser in eine Trägerflüssigkeit erzeugt werden. Dieses Vorgehen wird insbesondere in der automatisierten Prozessanalyse praktiziert, bei der eine eine Dialysemembran aufweisende Tauchsonde in das Wasser eingetaucht ist. Eine Trägerflüssigkeit nimmt das Analyt aus dem Wasser durch die Dialysemembran hindurch auf, und wird auf diese Weise zur Flüssigkeitsprobe, der anschließend durch eine Pumpe das Reagenz in genauer Dosierung zugeführt wird.

Alternativ oder ergänzend zur Ermittlung des Mischungsverhältnisses kann die Konzentration des Reagenz in der Flüssigkeitsprobe aus der Farbstandard-Absorption ermittelt werden. Auf diese Weise kann die Offset-Absorption des Reagenz bestimmt werden, die ebenfalls für die genaue Ermittlung der Konzentration des Analyts in der Flüssigkeitsprobe bekannt sein muss.

Im Folgenden werden zwei Ausführungsbeispiele der Erfindung näher erläutert.

In einem ersten Ausführungsbeispiel wird in einem Prozessanalyse-Gerät ein Anolyt in Wasser, beispielsweise in Abwasser, automatisch und quasikontinuierlich fotometrisch quantitativ bestimmt. Das zu bestimmende Anolyt ist beispielsweise Phosphat, es können jedoch auch grundsätzlich andere Moleküle bzw. Ionen in dem Wasser bestimmt werden.

Das Prozessanalyse-Gerät weist eine Tauchsonde mit einer Dialysemembran auf, die in Kontakt mit dem zu untersuchenden Wasser steht. Innenseitig fließt an der Dialysemembran eine Trägerflüssigkeit vorbei, so dass das Analyt durch die Dialysemembran hindurch in die langsam vorbei fließende Trägerflüssigkeit wandert. Die auf diese Weise gewonnene Flüssigkeitsprobe wird durch eine Pumpe zu einer Fotometer-Messstrecke gepumpt. Auf dem Weg dahin wird durch eine weitere Pumpe ein flüssiges Reagenz in den Flüssigkeitsproben-Strom eingeleitet.

Dem Reagenz ist ein inerter Farbstandard bekannter Konzentration zugefügt. Das Reagenz reagiert mit dem Analyt in der Flüssigkeitsprobe bei einer Reagenz-Absorptionswellenlänge λ_{R} und der Farbstandard absorbiert bei einer Absorptionswellenlänge λ_{F}. Die Absorptionsspektren des Analyts und des Farbstandards interferieren nicht bei der Absorptionswellenlänge des Farbstandards beziehungsweise des Analyts. Vorliegend kann beispielsweise als Phosphat-Reagenz Molybdat-Vanadat verwendet werden, das mit Säure-Fuchsin als inerten Farbstandard versetzt ist. Aus der Reaktion des Reagenzes mit dem Analyt geht eine intensiv gelb gefärbte Komplexverbindung hervor, die mit blaue Licht der Wellenlänge λ_{R} = 320 bis 400 nm gut detektiert werden kann. Allerdings weist das vorgenannte Reagenz auch ohne Reaktion bereits eine Absorption im vorgenannten Wellenlängen-Bereich auf, weist also eine Offset-Absorption auf. Der rote Farbstandard Säure-Fuchsin kann vorlegend bei einer Wellenlänge λ_{F}= 525 nm detektiert werden.

In dem Fotometer des Prozessanalysegerätes wird die mit dem Reagenz beaufschlagte Wasserprobe bei mindestens zwei Wellenlängen fotometrisch untersucht, und zwar bei der Farbstandard-Absorptionswellenlänge λ_{F} = 525 nm und bei einer Reagenz-Absorptionswellenlänge λ_{R} zwischen 320 und 400 nm.

Aus der fotometrischen Absorption der Flüssigkeitsprobe bei der Farbstandard-Absorptionswellenlänge λ_{F} lässt sich das genaue Mischungsverhältnis der Flüssigkeitsprobe mit dem flüssigen Analyt ermitteln. Hieraus wiederum lässt sich zum einen die Offset-Absorption des Reagenzes ermitteln. Zum anderen lässt sich die Netto-Absorption, also die Gesamt-Absorption des Reagenzes abzüglich der Offset-Absorption, ermitteln, da das genaue Mischungsverhältnis der Wasserprobe und des flüssigen Reagenzes bekannt ist. Schfießiich lässt sich ferner überprüfen, ob das Reagenz in einem ausreichenden Überschuss in der Wasserprobe vorliegt.

Bei einer relativ starken Abweichung der Farbstandard-Absorption von einem Sollwert kann angenommen werden, dass sich in der Fotometer-Messstrecke Luftblasen gebildet haben, die die Reagenz-Absorption stark verfälschen können. Bei einer starken Abweichung von dem Farbstandard-Sollwert wird die Messung der Reagenz-Absorption daher verworfen und gegebenenfalls ein entsprechendes Störungssignal ausgegeben.

Bei einem zweiten Ausführungsbeispiel handelt es sich um manuelle Labor-Fotometrie. Hierbei befindet sich das flüssige Reagenz in bekannter Menge und Konzentration bereits in einer Küvette, die herstellerseitig fertig konfektioniert beziehbar ist. In die Küvette wird manuell eine Wasserprobe pipettiert. Anschließend wird die Küvette in ein Fotometer gestellt, in dem die Absorption sowohl bei der Farbstandard-Absorptionswellenlänge λ_{F} als auch bei einer Reagenz-Wellenlänge λ_{R} bestimmt wird. Aus der Farbstandard-Absorption wird in oben beschriebener Weise in dem Fotometer die Offset-Absorption des Reagenzes ermittelt und die Normierung der Netto-Reagenz-Absorption vorgenommen. Da Luftblasen in der Wasserprobe bei diesem Verfahren in der Regel keine Rolle spielen, kann hierbei auf eine entsprechende Luftblasen-Überwachung verzichtet werden.

## Patentansprüche

1. Fotometrisches Verfahren zur quantitativen Bestimmung eines Analyts in Wasser, mit den Verfahrensschritten :
Erzeugen einer definierten Menge einer das Analyt enthaltenden Flüssigkeitsprobe,
Mischen einer definierten Menge eines Reagenzes mit der Flüssigkeitsprobe, wobei das Reagenz bei einer Reagenz-Absorptionswellenlänge λ_{R} farbverändernd mit dem Analyt reagiert,
Fotometrieren der Flüssigkeitsprobe in einem Fotometer bei der Reagenz- Absorptionswellenlänge λ_{R}, und
Ausgeben einer aus dem Ergebnis der Fotometrie ermittelten Analytkonzentration durch das Fotometer,
**gekennzeichnet durch**,
einen dem Reagenz zugefügten inerten Farbstandard bekannter Konzentration, wobei der Farbstandard eine charakteristische Absorbtionswellenlänge λ_{F} aufweist und das Reagenz-Absorbtionsspektrum und das Farbstandard- Absorbtionsspektrum so weit auseinander liegen, dass sie sich bei den jeweiligen Absorbtionswellenlängen λ_{R}, λ_{F} nicht überlagern,
Fotometrieren der Flüssigkeitsprobe mit dem Fotometer bei der Farbstandard- Absorptionswellenlänge λ_{F}, und
Ermitteln der fotometrischen Absorption der Flüssigkeitsprobe bei der Farbstandard- Absorptionswellenlänge λ_{F}-

2. Fotometrisches Verfahren gemäß Anspruch 1, mit dem Verfahrensschritt: Erzeugen der Flüssigkeitsprobe durch Isolieren einen Wasserprobe aus dem Wasser in ein Gefäß.

3. Fotometrisches Verfahren gemäß Anspruch 1, mit dem Verfahrensschritt: Erzeugen der Flüssigkeitsprobe durch Dialyse des Analyts aus dem Wasser in eine Trägerflüssigkeit.

4. Fotometrisches Verfahren gemäß einem der vorangegangenen Ansprüche, mit dem Verfahrensschritt: Ermitteln des Mischungsverhältnisses des flüssigen Reagenzes und der Flüssigkeitsprobe aus der Farbstandard-Absorption durch das Fotometer.

5. Fotometrisches Verfahren gemäß einem der vorangegangenen Ansprüche, mit dem Verfahrensschritt: Ermitteln der Offset-Absorption des Reagenzes in der Flüssigkeitsprobe aus der Farbstandard-Absorption durch das Fotometer,

6. Fotometrisches Verfahren gemäß einem der vorangegangenen Ansprüche, mit dem Verfahrensschritt: Ausgabe eines Störungssignales durch das Fotometer, wenn die Absorption der Flüssigkeitsprobe bei der Farbstandards- Absorptionswellenlänge nicht innerhalb eines feststehenden Intervalls liegt.

7. Fotometrisches Verfahren nach einem der vorangegangenen Ansprüche, **dadurch gekennzeichnet, dass** die Fotometrierung bei den beiden Wellenlängen λ_{F} und λ_{R} in derselben Mischung, in derselben Küvette und in demselben Fotometer erfolgt.

8. Fotometrisches Verfahren nach einem der vorangegangenen Ansprüche, **dadurch gekennzeichnet, dass** das Abmessen der definierten Menge des Reagenz durch einen Hersteller in fertig konfektionierten und einmal zu verwendenden Reagenziensätzen erfolgt.

9. Fotometrisches Verfahren nach einem der vorangegangenen Ansprüche, **dadurch gekennzeichnet, dass** das Abmessen und Mischen der definierten Mengen von Flüssigkeitsprobe und Reagenz automatisch in einem Prozessanalysegerät erfolgt.

10. Fotometrisches Verfahren nach einem der vorangegangenen Ansprüche, **dadurch gekennzeichnet, dass** eine Pumpe zum Abmessen und Mischen der definierten Mengen von Flüssigkeitsprobe und Reagenz benutzt werden.

11. Fotometrisches Verfahren nach einem der vorangegangenen Ansprüche, **dadurch gekennzeichnet, dass** das Reagenz ein in einer Küvette fertig dosierter Feststoff ist.
